# EUROPEAN PATENT APPLICATION

(11) **EP 3 056 508 A1**
(43) Date of publication of application: **17.08.2016**
(21) Application number: 15382048.5
(22) Date of filing: 10.02.2015
(51) Int. Cl.: C07H 17/04, A61K 31/7048, A61P 35/00

(54) **PI3K INHIBITOR COMPOUNDS TO TREAT CANCER**

(71) Applicant: FUNDACIÓN MEDINA. Centro de Excelencia en Investigación de Medicamentoslnnovadores en Andalucía, 18016 Armilla-Granada (ES)
(72) Inventor: De Pedro Montejo, Nuria, 28703 Madrid (ES); González Menéndez, Víctor, 18007 Granada (ES); Crespo Sueiro, Gloria, 28029 Madrid (ES); Cautain, Bastien, 18100 Armilla- Granada (ES); Fernández Acero, Teresa, 28035 Madrid (ES); Jiménez Cid, Víctor, 28224 Pozuelo de Alarcón-Madrid (ES); Molina Martín, María, 28035 Madrid (ES); Vicente Pérez, María Francisca, 28020 Madrid (ES); Reyes Benítez, José Fernando, 18150 Gójar- Granada (ES); Martín Serrano, Jesús Melchor, 18008 Granada (ES); Pérez-Victoria Moreno de Barreda, Ignacio, 18008 Granada (ES); Genilloud Rodríguez, Olga, 28006 Madrid (ES)
(74) Representative: ABG Patentes, S.L.

(57) **Abstract**

The present invention relates to compounds of general formula (I) 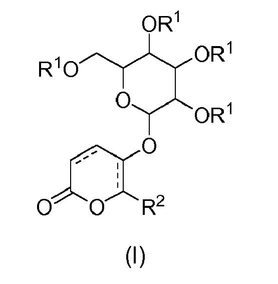 wherein R¹, R² and -̅ -̅ -̅ -̅ -̅ take various meanings, pharmaceutical compositions containing them and their use in medicine, particularly for the treatment and/or prophylaxis of cancer.

## Description

### FIELD OF THE INVENTION

The present invention relates to new compounds, pharmaceutical compositions containing them and their use in medicine, particularly as agents able to treat and/or prevent cancer. The present invention also relates to processes for preparing such compounds and compositions.

### BACKGROUND OF THE INVENTION

Phosphoinositide 3-kinases (PI3Ks, also known as phosphatidylinositol-3-kinases) are a family of related intracellular signal transducer enzymes capable of phosphorylating the hydroxyl group at position 3 of the inositol ring of phosphatidylinositol and have been linked to an extraordinarily diverse group of cellular functions.

PI3K activation activates AKT which activates mTOR and this signal cascade is known as PI3K/AKT/mTOR pathway.

It has been established the central role of PI3K signalling in several cellular processes, critical for cancer progression, including metabolism, growth, survival, and motility. Inappropriate co-option of PI3K signalling is one of the most frequent occurrences in human cancer. It is remarkable in this respect that mutations of the p110α catalytic subunit of Class I PI3K were found in 27% of breast tumors, 24% of endometrial tumors, 17% of urinary tumors and 15% of colon tumors; p110α amplifications were found in 69% of cervical tumors, 53% of squamous cell lung tumors, 36% of gastric tumors and 32% of head and neck tumors. In the last years, NSCLC (Non small cell lung cancer) is also likely to emerge as a therapeutic area of interest for PI3K inhibitors (Oncology business review. July 2011).

Inhibition of PI3K signalling can diminish cell proliferation, and in some circumstances, promote cell death. Accordingly, significant efforts have been made to generate inhibitors of the PI3K/AKT/mTOR pathway to treat different cancers and a great number of inhibitors have been developed and are being evaluated in preclinical studies and in early clinical trials: Inhibitors of PI3K (BEZ235, BGT226, BKM120, XL765, GDC09041, SF1126, GSK1059615), AKT inhibitors (Perifosine, MK2206, VQD-002, XL418), mTOR inhibitors (Rapamycin, CCI-779).

In spite of this pipeline, new PI3K/AKT/mTOR pathway inhibitors present both a great therapeutic opportunity and a tremendous challenge for cancer therapy.

### BRIEF DESCRIPTION OF THE INVENTION

The present invention solves the aforementioned need by the provision of new compounds able to inhibit the PI3K/AKT/mTOR pathway.

In one aspect, the present invention is directed to compounds of general formula (I) or pharmaceutically acceptable salts, stereoisomers or solvates thereof 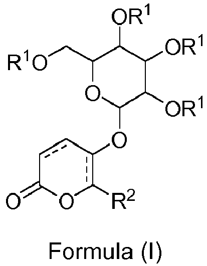 wherein
each R¹ is independently selected from hydrogen, substituted or unsubstituted alkyl, substituted or unsubstituted alkenyl, substituted or unsubstituted alkynyl and substituted or unsubstituted acyl;
R² represents a substituted or unsubstituted alkyl; and
each -̅ -̅ -̅ -̅ -̅ independently represent a single or double bond.

Another aspect of this invention refers to a medicament or pharmaceutical composition comprising at least one compound of formula (I) as defined above, or a pharmaceutically acceptable salt, stereoisomer, prodrug or solvate thereof and a pharmaceutically acceptable excipient.

Another aspect of this invention refers to a compound of formula (I) as defined above, or a pharmaceutically acceptable salt, stereoisomer or solvate thereof for use in medicine, particularly for the treatment and/or prophylaxis of cancer.

Another aspect of this invention refers to the use of a compound of formula (I) as defined above, or a pharmaceutically acceptable salt, stereoisomer or solvate thereof in the manufacture of a medicament for the treatment and/or prophylaxis of cancer.

Another aspect of the present invention refers to a method for the treatment and/or prophylaxis of cancer, the method comprising administering to the subject in need of such a treatment or prophylaxis an effective amount of a compound of formula (I) as defined above, or a pharmaceutically acceptable salt, stereoisomer or solvate thereof.

Thus, the present invention establishes that compounds of general formula (I) or pharmaceutically acceptable salts, stereoisomers or solvates thereof inhibit the PI3K/AKT/mTOR pathway. The compounds of the invention have been found to exhibit inhibition activity in the PI3K/AKT/mTOR pathway for different cancer treatments.

The present invention also relates to the obtention of compounds of formula (I) from microorganisms capable of producing them, specifically from a fungus identified as *Abortiporus biennis,* and more specifically from the strain of *A. biennis* CBS 137645, and the formation of derivatives from the obtained compounds. In a preferred embodiment, the process for obtaining compounds of formula (I) comprises the steps of cultivating a strain of a microorganism capable of producing them such as *A. biennis* CBS 137645 in an aqueous nutrient medium with assimilable carbon and nitrogen sources and salts, under controlled submerged aerobic conditions, and then recovering and purifying the compounds according to the invention from the culture broth.

Another aspect of the invention is the strain of *Abortiporus biennis* with accession number CBS 137645.

These aspects and preferred embodiments thereof are additionally also defined hereinafter in the detailed description, as well as in the claims.

### BRIEF DESCRIPTION OF THE FIGURES

**Figure 1****.** The figure shows percentage of cells in each cell cycle phase after treatment of MCF-7 cell line with different concentrations of MDN-0088.
**Figure 2****.** The figure shows a dose-response curve of MDN-0088 after a 72-hour treatment of the MCF-7 cell line.

### DETAILED DESCRIPTION OF THE INVENTION

Cancer is a leading cause of human death, and it is fundamentally attributable to dysfunctional cell signaling. The PI3K/AKT/mTOR pathway is an important pro-growth intracellular signaling cascade that is often inappropriately activated in a wide array of cancers. Efforts to develop anticancer drugs have therefore focused, in great part, on identifying PI3K/AKT/mTOR pathway inhibitors.

The present invention relates to novel compounds able to treat and/or prevent cancer. These compounds, of the general formula (I), are particularly useful as inhibitors of the PI3K/AKT/mTOR pathway.

The term "acyl", as used herein, refers to a group of structure -C(O)R, wherein R is substituted or unsubstituted alkyl (i.e. alkylacyl) or substituted or unsubstituted aryl (i.e. arylacyl).

As used herein by "substituted" is meant that the group in question may be substituted at one or more available positions by one or more suitable groups such as OR', =O, SR', =S, NHR', N(R')₂, and halogen, wherein each of the R' groups is independently selected from the group consisting of hydrogen, substituted or unsubstituted C₁-C₆ alkyl and substituted or unsubstituted C₆-C₁₀ aryl.

The compounds of general formula (I) comprise the following sugar moiety:

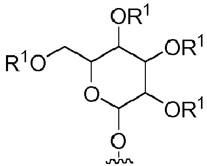

According to the invention, the sugar moiety may be chosen equally well from the laevorotatory sugar series (L-series) or dextrorotatory sugar series (D-series), but are chosen more particularly from the dextrorotatory sugar series (D-series).

The open-chain form of sugar moiety typically exists in equilibrium with several cyclic isomers. Both, the open-chain and the cyclic forms, are contemplated for the present invention, while preferably the sugar moiety is present as a cyclic form and more preferably as a pyranose (six-membered) ring.

The linkage between the sugar moiety and the rest of the molecule is a covalent glycosidic bond between the reducing group (hydroxyl) of the alcohol functional group of the hemiacetal carbon of a sugar (anomeric carbon, number 1) and the acid group (free hydrogen) of the rest of the molecule. This bond may equally well be of the alpha or beta type, and preferably of the beta type.

In the compounds of the present invention, preferably each R¹ is independently selected from hydrogen, substituted or unsubstituted C₁-C₆ alkyl, substituted or unsubstituted C₂-C₆ alkenyl, substituted or unsubstituted C₂-C₆ alkynyl, substituted or unsubstituted (C₁-C₆ alkyl)acyl and substituted or unsubstituted benzoyl.

More preferably each R¹ is independently selected from hydrogen, substituted or unsubstituted methyl, substituted or unsubstituted ethyl, substituted or unsubstituted n-propyl, substituted or unsubstituted iso-propyl, substituted or unsubstituted n-butyl, substituted or unsubstituted tert-butyl, substituted or unsubstituted vinyl, substituted or unsubstituted allyl, substituted or unsubstituted ethynyl, substituted or unsubstituted acetyl and substituted or unsubstituted benzoyl.

Even more preferably each R¹ is independently selected from hydrogen, acetyl, benzyl and benzoyl.

Even still more preferably R¹ represents hydrogen, that is, the sugar moiety in general formula (I) is glucose. More particularly, the sugar moiety is D-glucose, and even more particularly β-D-glucopyranose.

In the compounds of the present invention, R² is an alkyl optionally substituted. In a more preferred embodiment, the alkyl chain R² has from 1 to about 18 carbon atoms, such as from 1 to about 15 carbon atoms. One more preferred class of alkyl groups has from 8 to about 14 carbon atoms; even more preferred are alkyl groups having 9, 10, 11, 12 or 13 carbon atoms. In a more preferred embodiment, R² is an alkyl group optionally substituted having 11 carbon atoms.

If substituted, preferably R² is substituted at one position by one group selected from OR', =O, =S, SR', NHR', N(R')₂, and halogen, wherein each of the R' groups is independently selected from the group consisting of hydrogen, substituted or unsubstituted C₁-C₆ alkyl and substituted or unsubstituted C₆-C₁₀ aryl.

If substituted, more preferably R² is substituted at one position by =O, -OH or - NH₂. Besides, more preferably R² is substituted at carbon 9 (i.e. the fourth carbon if numbering starts from the carbon attached to rest of the molecule).

Particular examples of R² are the following:

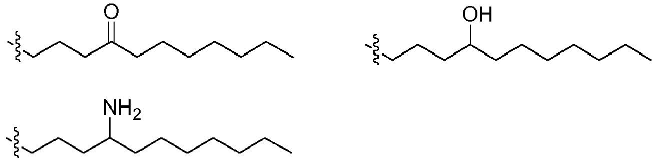

According to a preferred embodiment, R² is the following alkyl chain:

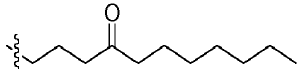

In a preferred embodiment of the invention, both -̅ -̅ -̅ -̅ -̅ represent simultaneously a double bond.

The term "salt" is to be understood as meaning any form of the active compound according to the invention in which this assumes an ionic form or is charged and is coupled with a counter-ion (a cation or anion) or is in solution. By this are also to be understood complexes of the active compound with other molecules and ions, in particular complexes which are complexed via ionic interactions. The definition includes in particular physiologically acceptable salts; this term must be understood as equivalent to "pharmacologically acceptable salts" or "pharmaceutically acceptable salts".

The term "physiologically acceptable salt" or "pharmaceutically acceptable salt" is understood in particular, in the context of this invention, as a salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid, picric acid and/or aspartic acid. Examples of physiologically tolerated salts of particular bases are salts of alkali metals and alkaline earth metals and with NH₄.

For those persons skilled in the art, it will be evident that the scope of the present invention also includes salts which are not pharmaceutically acceptable as possible means for obtaining pharmaceutically acceptable salts.

The term "solvate" according to this invention is to be understood as meaning any form of the compound according to the invention in which this compound has attached to it via non-covalent binding another molecule (most likely a polar solvent) especially including hydrates and alcoholates, e.g. methanolate. Methods of solvation are generally known within the art. The compounds of the invention may present different polymorphic forms, and it is intended that the invention encompasses all such forms.

Any compound referred to herein is intended to represent such specific compound as well as certain variations or forms. The compounds of the present invention represented by the above described formula (I) include stereoisomers. The term "stereoisomer" as used herein includes any enantiomer, diastereomer or geometric isomer (E/Z) of such compound. In particular, compounds referred to herein may have asymmetric centres and therefore exist in different enantiomeric or diastereomeric forms. Thus any given compound referred to herein is intended to represent any one of a racemate, one or more enantiomeric forms, one or more diastereomeric forms, and mixtures thereof. Likewise, stereoisomerism or geometric isomerism about the double bond is also possible, therefore in some cases the molecule could exist as (E)-isomer or (Z)-isomer (trans and cis isomers). If the molecule contains several double bonds, each double bond will have its own stereoisomerism, that could be the same or different than the stereoisomerism of the other double bonds of the molecule. All the stereoisomers including enantiomers, diastereoisomers and geometric isomers of the compounds referred to herein, and mixtures thereof, are considered within the scope of the present invention.

Furthermore, any compound referred to herein may exist as tautomers. Specifically, the term tautomer refers to one of two or more structural isomers of a compound that exist in equilibrium and are readily converted from one isomeric form to another. Common tautomeric pairs are enamine-imine, amide-imidic acid, keto-enol, lactam-lactim, etc.

Unless otherwise stated, the compounds of the invention are also meant to include isotopically-labelled forms i.e. compounds which differ only in the presence of one or more isotopically-enriched atoms. For example, compounds having the present structures except for the replacement of at least one hydrogen atom by a deuterium or tritium, or the replacement of at least one carbon by ¹³C- or ¹⁴C-enriched carbon, or the replacement of at least one nitrogen by ¹⁵N-enriched nitrogen are within the scope of this invention.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

To provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

A particularly preferred compound of the invention is the following: 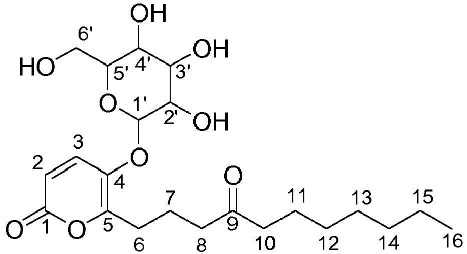 or a pharmaceutically acceptable salt, stereoisomer or solvate thereof.

Even a more preferred particular compound of the invention is the following, referring herein to as MDN-0088: 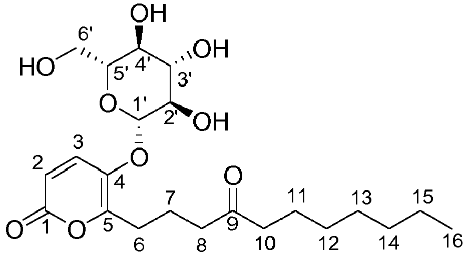 or a pharmaceutically acceptable salt or solvate thereof.

In additional preferred embodiments, the preferences described above for the different groups and substituents in the formulae above are combined. The present invention is also directed to such combinations.

The compound MDN-0088 was isolated from a fungus identified as *Abortiporus biennis.* The fungus is deposited in the culture collection of Fundación MEDINA under the accession number CF-080365 and has been also deposited under the Budapest Treaty in culture collection of the Centraalbureau voor Schimmelcultures, Uppsalalaan 8, 3584 CT Utrecht, the Netherlands with accession number CBS 137645.

One aspect of the present invention is said strain of *Abortiporus biennis* deposited at the CBS with accession number CBS 137645 as well as a culture thereof including a biologically pure culture. A description of this microorganism is provided hereinafter in the experimental part.

Thus, the antitumoral compounds of the invention may be produced by cultivating the strain *Abortiporus biennis* CF-080365, CBS 137645 in a suitable nutrient medium, such as those described below, until a significant amount accumulates in the fermentation.

The strain is usually cultured in an aqueous nutrient medium containing sources of assimilable carbon and nitrogen. For example, the cultures may be grown under submerged aerobic conditions (e.g., shaking the culture, submerging the culture, etc.) or in solid state fermentations. The aqueous medium is preferably maintained at a pH of about 6-8, at the initiation and termination (harvest) of the fermentation process. The desired pH may be maintained by the use of a buffer, such as morpholinoethane-sulfonic acid (MES), morpholinopropane-sulfonic acid (MOPS), and the like, or by choosing nutrient materials that inherently possess buffering properties. Suitable sources of carbon in the nutrient medium include carbohydrates, such as glucose, xylose, galactose, glycerine, starch, sucrose, dextrin and the like. Other suitable carbon sources that may be used include maltose, rhamnose, raffinose, arabinose, mannose, sodium succinate and the like. Suitable sources of nitrogen are yeast extracts, meat extracts, peptone, gluten meal, cottonseed meal, soybean meal and other vegetable meals (partially or totally defatted), casein hydrolysates, soybean hydrolysates, yeast hydrolysates, corn steep liquor, dried yeast, wheat germ, feather meal, peanut powder, distiller's solubles and the like, as well as inorganic and organic nitrogen compounds, such as ammonium salts (including ammonium nitrate, ammonium sulfate, ammonium fosfate, etc.), urea, amino acids, and the like. The carbon and nitrogen sources, which may be advantageously employed in combination, do not need be used in their pure forms; because less pure materials, which contain traces of growth factors, vitamins and significant quantities of mineral nutrients, are also suitable for use. In the case it would be necessary, mineral salts, such as sodium or calcium carbonate, sodium or potassium fosfate, sodium or potassium chloride, sodium or potassium iodide, magnesium salts, copper salts, cobalt salts, and the like, may be added to the medium. If necessary, especially when a culture medium foams excessively, one or more defoaming agent(s), such as liquid paraffin, fatty oils, plant oils, mineral oils or silicones, may be added. Submerged aerobic cultural conditions are typical methods of culturing cells for the production of cells in massive amounts. For small-scale production, a shaken or surface culture in a flask or bottle may be employed. When growth is carried out in large tanks, it may be preferable to use the vegetative forms of the organism for inoculation in the production tanks in order to avoid growth lag in the process of production. Accordingly, it may be desirable first to produce a vegetative inoculum of the organism by inoculating a relatively small quantity of culture medium with spores or mycelia of the organism produced in a "slant" or Petri dish and culturing said inoculated medium, also called the "seed medium," and then to transfer the cultured vegetative inoculum aseptically to large tanks. The fermentation medium, in which the inoculum is produced, is generally autoclaved to sterilize the medium prior to inoculation. The pH of the medium is generally adjusted to about 6-7 prior to the autoclaving step. Agitation and aeration of the culture mixture may be accomplished in a variety of ways. Agitation may be provided by a propeller or similar mechanical agitation equipment, by revolving or shaking the fermentor, by various pumping equipment, or by the passage of sterile air through the medium. The fermentation is usually conducted at a temperature between about 20 °C and 30 °C, such as between about 22 °C and 25 °C, for a period of about 7 to 21 days, and parameters may be varied according to fermentation conditions and scales. Preferred culturing/production media for carrying out the fermentation include the media as set forth in the examples. Culture media such as MV8 or YES are particularly preferred.

Separation and purification of compounds of the present invention from the crude active extract can be performed using the proper combination of conventional chromatographic techniques.

Additionally, compounds of the invention can be obtained by modifying those already obtained from the natural source or by further modifying those already modified by using a variety of chemical reactions, for instance by means of derivatization, protection/deprotection and isomerisation reactions. Thus, hydroxyl groups can be acylated by standard coupling or acylation procedures, for instance by using acetic acid, acetyl chloride or acetic anhydride in pyridine or the like. Formate groups can be obtained by heating hydroxyl precursors in formic acid. Carbamates can be obtained by heating hydroxyl precursors with isocyanates. Hydroxyl groups can be converted into halogen groups through intermediate sulfonates for iodide, bromide or chloride, or directly using a sulfur trifluoride for fluorides; or they can be reduced to hydrogen by reduction of intermediate sulfonates. Hydroxyl groups can also be converted into alkoxy groups by alkylation using an alkyl bromide, iodide or sulfonate, or into amino lower alkoxy groups by using, for instance, a protected 2-bromoethylamine. Amide groups can be alkylated or acylated by standard alkylation or acylation procedures, for instance by using, respectively, KH and methyl iodide or acetyl chloride in pyridine or the like. Ester groups can be hydrolized to carboxylic acids or reduced to aldehyde or to alcohol. Carboxylic acids can be coupled with amines to provide amides by standard coupling or acylation procedures. When necessary, appropriate protecting groups can be used on the substituents to ensure that reactive groups are not affected. The procedures and reagents needed to prepare these derivatives are known to the skilled person and can be found in general textbooks such as March's Advanced Organic Chemistry 6th Edition 2007, Wiley Interscience. As the skilled person will appreciate, certain compounds of formula (I) may be useful as intermediate products in the preparation of other compounds of formula (I).

An important feature of the above described compounds is their bioactivity and in particular their activity as inhibitors of the PI3K/AKT/mTOR pathway and therefore their utility in cancer therapy. Thus, a further aspect of the present invention relates to a medicament or composition in different pharmaceutical forms comprising at least a compound of formula (I), optionally at least another anticancer drug and at least one pharmaceutically acceptable excipient for use in the treatment and/or prevention of cancer.

Examples of pharmaceutical compositions include any solid (tablets, pills, capsules, granules, etc.) semi-solid (creams, ointments, etc.) or liquid (solutions, suspensions or emulsions) composition for oral, topical or parenteral (such as intravenous) administration.

The respective medicament may - depending on its route of administration - also contain one or more excipients known to those skilled in the art. The medicament according to the present invention may be produced according to standard procedures known to those skilled in the art.

The term "excipient" refers to components of a drug compound other than the active ingredient (definition obtained from the European Medicines Agency- EMA). They preferably include a "carrier, adjuvant and/or vehicle". Carriers are forms to which substances are incorporated to improve the delivery and the effectiveness of drugs. Drug carriers are used in drug-delivery systems such as the controlled-release technology to prolong *in vivo* drug actions, decrease drug metabolism, and reduce drug toxicity. Carriers are also used in designs to increase the effectiveness of drug delivery to the target sites of pharmacological actions (*U.S. National Library of Medicine. National Institutes of Health*). Adjuvant is a substance added to a drug product formulation that affects the action of the active ingredient in a predictable way. Vehicle is an excipient or a substance, preferably without therapeutic action, used as a medium to give bulk for the administration of medicines (Stedman's Medical Spellchecker, @ 2006 Lippincott Williams & Wilkins). Such pharmaceutical carriers, adjuvants or vehicles can be sterile liquids, such as water and oils, including those of petroleum, animal, vegetable or synthetic origin, such as peanut oil, soybean oil, mineral oil, sesame oil and the like, excipients, disgregants, wetting agents or diluents. Suitable pharmaceutical carriers are described in "Remington's Pharmaceutical Sciences" by E.W. Martin. The selection of these excipients and the amounts to be used will depend on the form of application of the pharmaceutical composition.

As used herein, the terms "treat", "treating" and "treatment" include in general the eradication, removal, reversion, alleviation, modification, or control of cancer after its onset.

As used herein, the terms "prevention", "preventing", "preventive", "prevent" and prophylaxis refer to the capacity of a given substance, composition or medicament to avoid, minimize or difficult the onset or development of cancer before its onset.

The present invention also encompasses the combination of the compounds of formula (I) with other anticancer drug. A combination of at least a compound of formula (I) and at least another anticancer drug may be formulated for its simultaneous, separate or sequential administration. This has the implication that the combination of the two compounds may be administered:
- as a combination that is being part of the same medicament formulation, the two compounds being then administered always simultaneously.
- as a combination of two units, each with one of the substances giving rise to the possibility of simultaneous, sequential or separate administration.

In a particular embodiment, the compound of formula (I) is independently administered from the other anticancer drug (i.e in two units) but at the same time.

In another particular embodiment, the compound of formula (I) is administered first, and then the other anticancer drug is separately or sequentially administered.

In yet another particular embodiment, the other anticancer drug is administered first, and then the compound of formula (I) is administered, separately or sequentially, as defined.

Another aspect of the invention is a method of treatment of a patient, notably a human, suffering cancer, or likely to suffer cancer, which comprises administering to the patient in need of such a treatment or prophylaxis an effective amount of a compound of formula (I).

Examples or cancers on which the compounds of formula (I) of the invention may be used include, without limitation, cervical tumors, squamous cell lung tumors, gastric tumors, head and neck tumors and NSCLC (Non small cell lung cancer).

By an "effective" amount or a "therapeutically effective amount" of a drug or pharmacologically active agent is meant a nontoxic but sufficient amount of the drug or agent to provide the desired effect. In the therapy of the present invention, an "effective amount" of the compound of formula (I) is the amount of that compound that is effective to provide the desired effect. The amount that is "effective" will vary from subject to subject, depending on the age and general condition of the individual, the particular active agent or agents, and the like. Thus, it is not always possible to specify an exact "effective amount". However, an appropriate "effective" amount in any individual case may be determined by one of ordinary skill in the art using routine experimentation.

The following examples are merely illustrative of certain embodiments of the invention and cannot be considered as restricting it in any way.

### EXAMPLES

### EXAMPLE 1: DESCRIPTION OF FUNGAL STRAIN

The producer fungus (CF-080365; CBS 137645) was isolated from fruiting bodies collected in La Hiruela, Madrid, Spain. An axenic culture was obtained from the inner fragments of the fruit bodies living tissues using a malt extract isolation medium supplemented with benomyl and dichloran to inhibit the growth of non basidiomycetous fungi as described in Worrall (1991).

On oatmeal agar, the fungus presents initially whitish colonies that become pinkish after three weeks. Mycelium is cottony-woolly. After 4 to 6 weeks, the mycelium forms irregular iripicoid to poroid shaped structures, with a pinkish and a reddish exudate. After seven days, the presence of the anamorphic state is evident, conidiophores are hyaline, thin-walled, racemose, branched from clamp connections. Conidia are terminal, thick-walled, globose to subglobose. The growth attains 80-90 mm in radius in two weeks.

On 2% malt agar, the fungus presents whitish colonies at 3-4 weeks turning pink at the margins of the colony. The mycelia are cottony-woolly. After 4 to 6 weeks, the mycelium forms irregular iripicoid to poroid shaped structures in margins of the colony, with a reddish exudate. After three weeks the presence of anamorphic state is evident. Conidiophores are hyaline, thin-walled, racemosely branched from clamp connections. Conidia are terminal, thick-walled, globose to subglobose. The growth attains 80-90 mm in radius in two weeks.

On synthetic nutrient agar, the fungus presents whitish colonies, with little growth of the mycelium in the agar, forming aerial mycelia predominantly loose strands. The anamorphic state is not evident. The growth attains 80-90 mm in radius in three weeks.

To estimate the phylogenetic position of strain CF-080365, genomic DNA was extracted from mycelia grown on malt-yeast extract agar. The rDNA region containing the partial sequence of 28S rDNA containing D1 D2 variable domains was amplified with primers NL1 and NL4 (O'Donnell K, 1993. Fusarium and its near relatives. In: Reynolds DR, Tailor JW (eds)). The fungal holomorph: mitotic, meiotic and pleomorphic speciation in fungal systematic, CAB international, Wallingford, UK, pp. 225-233) and a DNA sequence was generated. About 0.1 µg/mL of the double-stranded amplification products were sequenced using the ABI PRISM Dye Terminator Cycle Sequencing Ready Reaction Kit (Perkin Elmer, Norwalk CT) following the procedures recommended by the manufacturer. Purified PCR products were directly sequenced using the same primer pairs as in the PCR reactions. Partial sequences obtained in sequencing reactions were assembled with Genestudio 2.1.1.5. (Genestudio, Inc., Suwanee, GA, USA). Database matching with the 28S rDNA sequence (www.fungalbar.org), yielded a very high sequence similarity (99%) to the strain of *Abortiporus biennis* voucher EL65-03, thus indicating that strain CF-080365 is genetically similar to *A. biennis,* and probably conspecific. High similar scores to other authentic fungi strains *Cymatoderma dendriticum* CBS 207.62, indicated that CF-080365 could be classified as *Abortipoprus biennis* (Basidiomycota, Polyporales, Meruliaceae).

### EXAMPLE 2: FERMENTATION IN ERLENMEYER FLASKS

The most potent activity from extracts of a medium designated as MV8 (maltose 75 g, V8 juice 200 mL, soy flour 1 g, L-proline 3 g, MES 16.2 g, 800 mL H₂O) was selected for further study. The initial sample for characterization of PI3K pathway inhibitor activity was prepared by cutting five mycelial discs from each 60-mm plate and crushing them in the bottom of tubes (25 x 150 mm) containing 12 mL of Sabouraud maltose broth supplemented with yeast extract and dilute agar (SMYA) (Difco neopeptone 10 g, maltose 40 g, Difco yeast extract 10 g, agar 4 g, distilled H₂O 1000 mL) and two cover glasses (22 x 22 mm). Tubes were agitated on an orbital shaker (220 rpm, 5 cm throw), to produce homogenous hyphal suspensions. After growing the inoculum stage for 7 days, a 0.3 mL aliquot was used to inoculate10 mL of MV8 medium in 2 x 10-mL EPA vials. The tubes were incubated in aerated agitated fermentation (220 rpm) for 21 days at 22 °C. Mycelium and broth from these tubes were extracted with acetone, and after removing the acetone by evaporation, the aqueous residue was used to detect a series of active molecules. To further characterize the molecule responsible for the activity, a 1-L fermentation was prepared. Ten mycelial discs were used to inoculate 50 mL of SMYA. After 7 days, 3-mL aliquots of this culture were used to inoculate MV8 medium (10 x 100 mL medium in 500-mL Erlenmeyer flasks). The flasks were incubated in an aerated agitated fermentation (220 rpm, 5 cm throw) at 22 °C, 70% relative humidity for 21 days.

### EXAMPLE 3: ISOLATION AND STRUCTURAL IDENTIFICATION OF MDN-0088

The fermentation broths (2L in flasks) were extracted with methyl ethyl ketone (2 x 2L). The organic phase was evaporated to dryness to afford 4.12 g of a dried extract that was subjected to reversed phase silica gel (RP18) chromatography (70g C18, 40-60µm). The column was washed with 10% of CH₃CN in water for 10min and eluted at 18 mL/min using a linear gradient from 10% to 100% CH₃CN in water for 35 min with a final 100% CH₃CN wash step of 19 min collecting 52 fractions of 18 mL. The compound of interest was detected to elute in the 52-53% CH₃CN fractions by HPLC-MS. These fractions were further purified by reversed phase semi-preparative HPLC (x-Bridge C18, 10 x 150 mm, 5 µm; 3.6 mL/min, UV detection at 210 nm and 310nm) with a linear gradient of CH₃CN in water from 30% to 50% over 35 min where compound MDN-0088 (11.7 mg) eluted at 15.25 min.

**MDN-0088:** white solid; [α]^{25.9}_{D} -15.1 (c=0.11, MeOH); UV (DAD) λₘₐₓ 315 nm; I R (ATR) γ cm⁻¹: 3390, 2926, 2857, 1710, 1633, 1548,1456, 1410, 1377, 1235, 1073, 1046. HRMS m/z 443.2274 [M+H]⁺ (calc. for C₂₂H₃₅O₉⁺, 443.2276, Δ -0.4ppm); 460.2547 [M+NH₄]⁺ (calc. for C₂₂H₃₈NO₉⁺, 460.2541, Δ +1.3ppm); ¹H and ¹³C NMR data see Table 1:

**Table 1. NMR Spectroscopic Data (500 MHz, CD₃OD) for MDN-0088.**

| **n°** | **δ_{C}, type** | **δ_{H}, mult**. **(*J* in HZ)** | **COSY** | **HMBC (H to C)** |
|---|---|---|---|---|
| 1 | 163.9, C | | | |
| 2 | 113.8, CH | 6.16, d(9.9) | 3 | 1,4,5 |
| 3 | 144.6, CH | 7.71, d (9.9) | 2 | 1,4,5 |
| 4 | 138.9, C | | | |
| 5 | 158.2, C | | | |
| 6 | 28.5, CH2 | 2.70, m | 7 | 4, 5, 7, 8 |
| 7 | 22.0, CH2 | 1.91, q (7.1) | 6, 8 | 5,6,8,9 |
| 8 | 42.4, CH2 | 2.55, t (7.1) | 7 | 6,7,9 |
| 9 | 213.5, CO | | | |
| 10 | 43.6, CH2 | 2.46, t (7.5) | 11 | 9,11,12 |
| 11 | 24.8, CH2 | 1.54, q (7.1) | 10, 12 | 9, 10, 12, 13 |
| 12 | 30.3, CH2 | 1.30, m | 11, 13 | |
| 13 | 30.2, CH2 | 1.30, m | 12,14 | |
| 14 | 32.9, CH2 | 1.30, m | 13, 15 | |
| 15 | 23.7, CH2 | 1.30, m | 14, 16 | |
| 16 | 14.4, CH3 | 0.90, t (7.0) | 15 | 14,15 |
| 1' | 105.9, CH | 4.53, d (7.1) | 2' | 4, 3', 5' |
| 2' | 74.9, CH | 3.36, m | 1' | 1',3' |
| 3' | 77.9, CH | 3.39, m | | 2', 4' |
| 4' | 71.2, CH | 3.34, m | | 3', 5', 6' |
| 5' | 78.4, CH | 3.29, m | 6' | 4' |
| 6' | 62.5, CH2 | 3.86, dd, (2.2, 12.1) | 5', 6'b | 4' |
| | | 3.69, dd, (5.5, 12.1) | 5', 6'a | 5' |

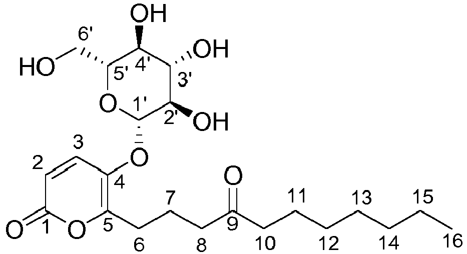

### EXAMPLE 4: DETERMINATION OF THE IDENTITY AND ABSOLUTE CONFIGURATION OF THE MONOSACCHARIDE UNIT OF COMPOUND MDN-0088

A coupling constant of 7.1 Hz observed for the anomeric proton H1' indicated an axial-axial coupling (corresponding to a β configuration), and therefore an axial orientation of H1' and H2'. NOESY correlations observed between H1' and H3' and H5' also indicated the axial orientation of the latter two protons. Finally, although coupling constants for H4' could not be measured due to overlapping of signals, the pattern observed in the TOCSY correlations obtained upon irradiation of the anomeric proton H1' (with 100msec of mixing time) confirmed its axial orientation and the monosacaride unit was unequivocally identified as β-glucopyranose.

To determine the absolute configuration, compound MDN-0088 was hydrolyzed in HCl 0.5N (3h at 90°C). The water soluble part of the hydrolizate was lyophilized and derivatized with methyl ester hydrochloride L-cysteine and o-tolyl isothiocyanate in pyridine at 60°C. D-glucose and L-glucose were derivatized in the same way and used as standards. The derivatized product and the standards were analyzed by HPLC-MS (x-Bridge C18, 4.6 x 150mm, 5µm, 35°C) with a linear gradient of CH₃CN in water (0.01%TFA and ammonium formate 1.3mM) from 5% to 50% over 15 min where derivatized D-glucose eluted at 13.44 min, derivatized L-glucose eluted at 13.18 min and the glycoside coming from the hydrolization of MDN-0088 eluted at 13.44 min.

The monosaccharide unit present in the structure of MDN-0088 was therefore identified as β-D-glucopyranose.

### EXAMPLE 5: ASSESSMENT OF CELL CYCLE INHIBITION ACTIVITY OF COMPOUND MDN-0088,

For the evaluation of the MDN-0088 activity, the MCF-7 cell line was used. The method used was flow cytometry to determine the effect of MDN-0088 on cell cycle. This method of DNA staining utilizes ethanol to fix the cells and permeabilize the membrane, which allows the dye (Propidium Iodide) to enter the cells.

For this purpose, cells were seeded in 6-well plates at 200,000 cells/well. After 24 hours, cells were serum-deprived for 72 hours. Medium was removed and compounds/standards were added in fresh medium at a different concentration (4µL of compound/2mL fresh medium). DMSO concentration was 0.2% in all wells. After 72 hours of incubation (37 °C, 5% CO₂), cells were scrapped and centrifuged (1200rpm for 5 min) and washed twice with PBS. 330 µL of cell suspension was placed in a Facs tube and 660 µL ice cold 100% ethanol was added drop-wise to the tube walls. Tubes were mixed by vortexing slowly during addition of ethanol. They were left at -20% overnight.

Tubes were centrifuged (1200 rpm for 5 min) to remove ethanol, 4 mL PBS was added and they were washed at 1200 rpm for 5 min. The supernatant was removed and cells were resuspended in DNA staining solution (500 µL PBS/IP 20µg/mL/Rnase 0.2 mg/ml). Data was acquired on a flow cytometer (Accury, BD).

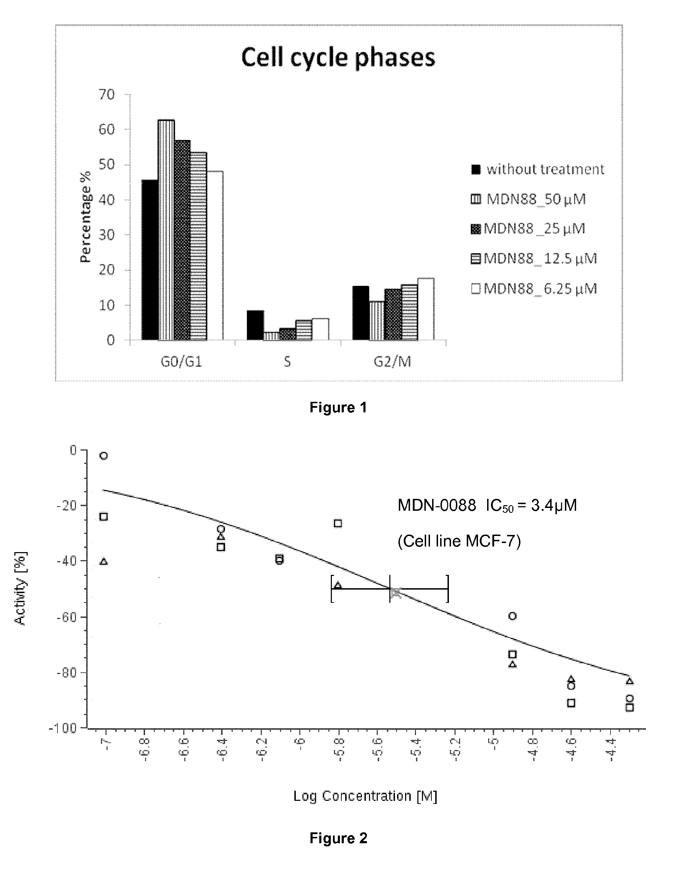

As shown in Table 2 and Figure 1, an increase in G0/G1 and a decrease both in S and G2/M phase were observed. Therefore, MDN-0088 blocks the cell cycle in G0/G1 phase.

**Table 2. The table shows the percentage of cells in each cell cycle phase.**

| | Percentage % | | |
|---|---|---|---|
| | **G0/G1** | **S** | **G2/M** |
| without treatment | 45.68 | 8.42 | 15.5 |
| MDN88 50 µM | 62.79 | 2.37 | 10.99 |
| MDN88 25 µM | 56.9 | 3.49 | 14.38 |
| MDN88_ 12.5 µM | 53.49 | 5.64 | 15.76 |
| MDN88_6.25 µM | 48.17 | 6.09 | 17.71 |

To evaluate the potency of compound MDN-0088 on cell proliferation, dye CFSE and High content screening (HCS) equipment were used. MCF-7 cell line was used. Cells were seeded in 96 wells plates at 10.000 cells/well. After 24 hours, medium was removed and compounds and standards were added in fresh medium at different concentration, (1 µL of compound/200 µL fresh medium), The DMSO concentration was 0.5% in all wells. Fluorescence in wells was read using a High Content Screening equipment each day during 5 days, to evaluate the effect of MDN-0088 and the IC₅₀was calculated (Figure 2).

### EXAMPLE 6: ASSESSMENT OF THE PI3K/AKT/mTOR PATHWAY INHIBITION ACTIVITY OF COMPOUND MDN-0088,

Western blot technique was used, using an Odissey Licor equipment to identify the proteins. Phospho AKT, AKT and Phospho GSK3β antibodies were used as first antibodies and anti-mouse 680nm as second antibody. Then, the signals were quantified using β-actine. Results are shown in Tables 3 and 4.

**Table 3. Results of AKT**

| Cells Treatment | AKT total |
|---|---|
| With insuline | 0.67 |
| Without insuline | 0.60 |
| MDN-0088_60 min | 0.58 |
| LY294002_60 min | 0.61 |

**Table 4. Results of p-AKT and p-GSK3β**

| | pAKT | phospho-GSK3 β |
|---|---|---|
| With insuline | 0.010 | 0.023 |
| Without insuline | 0.080 | 0.067 |
| MDN-0088_1 hour | 0.027 | 0.037 |
| LY294002_1 hour | 0.055 | 0.042 |

Table 3 shows that the concentration of total AKT does not change among different treatments used in the experiment, however as shown in Table 4 the concentration of pAKT and p-GSK3β decreases after treatment with MDN-0088.

### List of references:

- Oncology business review. July 2011.
- Worrall, J.J. (1991) Media for selective isolation of Hymenomycetes. Mycologia 83, 296-302.
- O'Donnell K, 1993. Fusarium and its near relatives. In: Reynolds DR, Tailor JW (eds).

## Claims

1. A compound of general formula (I) or pharmaceutically acceptable salt, stereoisomer or solvate thereof 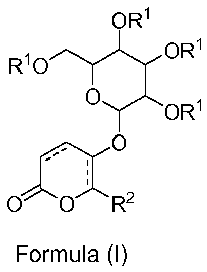 wherein
each R¹ is independently selected from hydrogen, substituted or unsubstituted alkyl. substituted or unsubstituted alkenyl, substituted or unsubstituted alkynyl and substituted or unsubstituted acyl;
R² represents a substituted or unsubstituted alkyl; and
each -̅ -̅ -̅ -̅ -̅ independently represent a single or double bond.

2. Compound according to claim 1, wherein each R¹ is independently selected from hydrogen, substituted or unsubstituted C₁-C₆ alkyl, substituted or unsubstituted C₂-C₆ alkenyl, substituted or unsubstituted C₂-C₆ alkynyl, substituted or unsubstituted (C₁-C₆ alkyl)acyl and substituted or unsubstituted benzoyl.

3. Compound according to claim 1, wherein each R¹ is independently selected from hydrogen, substituted or unsubstituted methyl, substituted or unsubstituted ethyl, substituted or unsubstituted n-propyl, substituted or unsubstituted iso-propyl, substituted or unsubstituted n-butyl, substituted or unsubstituted tert-butyl, substituted or unsubstituted vinyl, substituted or unsubstituted allyl, substituted or unsubstituted ethynyl, substituted or unsubstituted acetyl and substituted or unsubstituted benzoyl.

4. Compound according to claim 1, wherein each R¹ is independently selected from hydrogen, acetyl, benzyl and benzoyl.

5. Compound according to claim 1, wherein R¹ is hydrogen.

6. Compound according to any one of the preceding claims wherein R² is an alkyl group having 11 carbon atoms optionally substituted.

7. Compound according to any one of the preceding claims wherein R² is selected from one of the following alkyl chains:

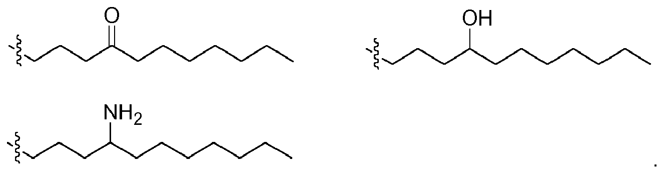

8. Compound according to any one of the preceding claims wherein R² is the following alkyl chain:

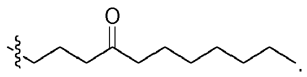

9. Compound according to any one of the preceding claims, which is: 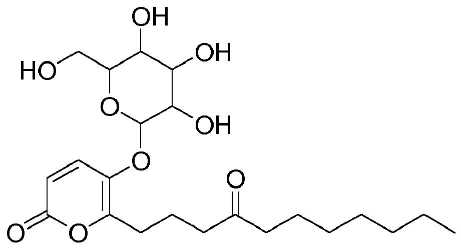 or a pharmaceutically acceptable salt, stereoisomer or solvate thereof.

10. Compound according to claim 9, which is: 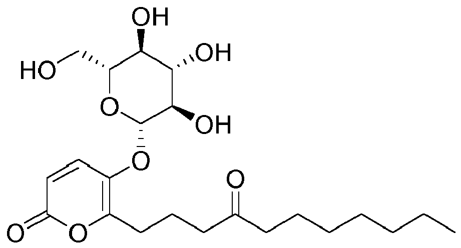 or a pharmaceutically acceptable salt or solvate thereof.

11. Pharmaceutical composition comprising at least one compound of formula (I) as defined in any one of claims 1 to 10, or a pharmaceutically acceptable salt, stereoisomer or solvate thereof and a pharmaceutically acceptable excipient.

12. Compound according to any one of claims 1 to 10 for use in medicine.

13. Compound according to any one of claims 1 to 10 for use in the treatment and/or prophylaxis of cancer.

14. A strain of *Abortiporus biennis* deposited at the CBS with accession number CBS 137645.

15. A process for obtaining a compound of general formula (I) as defined in any one of claims 1 to 10 or a pharmaceutically acceptable salt, stereoisomer or solvate thereof, said process comprising the steps of cultivating the strain of *Abortiporus biennis* CBS 137645 in an aqueous nutrient medium with assimilable carbon and nitrogen sources and salts, under controlled submerged aerobic conditions, and then recovering and purifying the compound of general formula (I) from the culture broth.
